(19) Europäisches Patentamt
European Patent Office
Office européen des brevets

(11) Veröffentlichungsnummer: **0 026 474**
**B1**

(12) # EUROPÄISCHE PATENTSCHRIFT

(45) Veröffentlichungstag der Patentschrift: **08.06.83**

(51) Int. Cl.³: **A 61 K 7/13**

(21) Anmeldenummer: **80105821.5**

(22) Anmeldetag: **25.09.80**

(54) **Mittel und Verfahren zur oxidativen Färbung von Haaren.**

(30) Priorität: **28.09.79 DE 2939304**

(43) Veröffentlichungstag der Anmeldung: **08.04.81 Patentblatt 81/14**

(45) Bekanntmachung des Hinweises auf die Patenterteilung: **08.06.83 Patentblatt 83/23**

(84) Benannte Vertragsstaaten: **DE GB IT SE**

(56) Entgegenhaltungen:
**DE-A-1 922 400**
**DE-A-2 334 738**
**DE-A-2 716 671**
**DE-A-2 719 179**
**DE-A-2 719 424**

(73) Patentinhaber: **Wella Aktiengesellschaft, Berliner Allee 65, D-6100 Darmstadt (DE)**

(72) Erfinder: **Bachmann, Heinrich, Gässli 300, CH-1711 Giffers (CH)**
Erfinder: **Portmann, Plato, Prof. Dr., Cité-Jardins, CH-1700 Fribourg (CH)**

EP 0 026 474 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Mittel und Verfahren zur oxidativen Färbung von Haaren

Es sind bereits Haarfärbemittel auf der Basis von Oxidationsfarbstoffen bekannt, welche dadurch gekennzeichnet sind, dass sie bestimmte Diaminobenzoxazol-, Diaminobenzofuran- bzw. Diaminobenzoxazolinonderivate sowie deren anorganische oder organische Salze als Kupplersubstanzen im Gemisch mit in Oxidationsfarben üblichen Entwicklersubstanzen enthalten. Diese Mittel liefern bei ihrer Anwendung auf dem Haar sehr intensive, von braun bis schwarzblau reichende Farbnuancen mit sehr guten Echtheitseigenschaften. Die erfindungsgemässen Kupplersubstanzen zeichnen sich durch eine gute Löslichkeit in Wasser, eine gute Lagerstabilität und toxikologische sowie dermatologische Unbedenklichkeit aus (vgl. DE-OS Nr. 2719424).

Ausserdem sind schon Haarfärbemittel bekannt geworden, die einen kennzeichnenden Gehalt an bestimmten 4-Hydroxychinolon-2-derivaten als Kupplersubstanzen aufweisen. Bei dem Einsatz dieser Kupplersubstanzen in Mischung mit verschiedenen üblichen Entwicklersubstanzen liefern die Haarfärbemittel die unterschiedlichsten Farbnuancen, die von hellblond bis dunkelbraun und grün über blau bis violett reichen. Die erzielten Färbungen haben gute Licht-, Wasch- und Reibechtheiten und lassen sich leicht mit Reduktionsmitteln wieder abziehen (vgl. DE-OS Nr. 2334738).

Zur Färbung von Haaren haben sogenannte Oxidationsfarbstoffe auf Grund ihrer Licht- und Waschechtheit eine wesentliche Bedeutung erlangt. Zur Erzeugung der Oxidationsfarbstoffe werden als Farbstoffvorstufen bestimmte, zu oxidativer Kupplung befähigte aromatische Verbindungen auf das Haar aufgebracht. Diese dringen teilweise in das Haar ein und werden dort entweder durch Luftsauerstoff oder insbesondere durch Zusatz chemischer Oxidationsmittel wie Wasserstoffperoxid zum gewünschten Farbstoff oxidiert.

Als Farbstoffvorstufen dienen hauptsächlich Derivate von Diamino- oder Hydroxyaminoverbindungen des Benzols, Naphthalins, Pyridins, Pyrimidins, Pyrazolons, Indols und Chinolins. Diese sind teilweise aus physiologischen Gründen nicht unbedenklich.

Ebenfalls bekannt als Farbstoffvorstufen sind Diphenol- und Naphtholderivate. Jedoch sind mit den bisher als Farbstoffvorstufen bekannten Vertretern dieser Substanzklassen keine intensiven Haarfärbungen erzielbar.

Weiterhin ist seit der Kenntnis der Bildung des natürlichen Haarpigmentes auch die Verwendung von Tyrosin, Dopa*) und Dihydroxyindolen zum Färben von Haar beschrieben worden.

Mittel auf der Basis dieser, aus physiologischer Sicht für eine Verwendung als Farbstoffvorstufen wohl günstigsten Verbindungen, konnten sich aus unterschiedlichen Gründen in der Praxis nicht durchsetzen.

---

*) Dopa=L-β-(3,4-Dihydroxyphenyl)alanin.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Ferner ist es erforderlich, dass durch Kombination von solchen, als Farbstoffvorstufen geeigneten Verbindungen, eine breite Palette verschiedener Farbnuancen erzeugt werden kann. Ausserdem wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben.

Der Vielzahl der gestellten Anforderungen können die zur Zeit in Oxidationshaarfärbemitteln als Farbstoffvorstufen verwendeten Verbindungen jedoch nicht völlig zufriedenstellend genügen.

Es bestand daher die Aufgabe, Mittel zur oxidativen Färbung von Haaren auf der Basis von solchen, als Farbstoffvorstufen dienenden Verbindungen zu erstellen, welche in physiologischer Hinsicht gegenüber den bekannten als Farbstoffvorstufen eingesetzten Verbindungen günstigere Eigenschaften besitzen. Dabei sollen sie allein oder in Kombination mit anderen als Farbstoffvorstufen bekannten Verbindungen gute Trageechtheiten und genügende Intensität der Färbungen aufweisen.

Erfindungsgegenstand ist daher ein Mittel zur oxidativen Färbung von Haaren, dadurch gekennzeichnet, dass es als Farbstoffvorstufe mindestens eine Verbindung der allgemeinen Formel

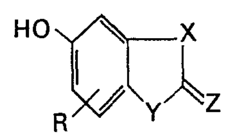

(I)

enthält, worin

X = $CH_2$, O, S, NH, NR', CH(OH), CHR', $CH(NH_2)$, $CH(CH_3)$,

Y = O, NH,

Z = O, S, NH,

R = H, OH, R', OR', $NR'_2$, $NO_2$, Halogen, und

R' = $C_1$ bis $C_5$-Alkyl

bedeuten.

Die Gesamtmenge dieser, als Farbstoffvorstufen dienenden Verbindungen gemäss Formel I soll in den Mitteln 0,1 bis 5 Gew.%, vorzugsweise 1 bis 4 Gew.%, betragen.

Beispiele für geeignete, in den erfindungsgemässen Haarfärbemitteln als Farbstoffvorstufen enthaltene Verbindungen der oben angegebenen allgemeinen Formel I sind insbesondere

1) 5-Hydroxy-[3H]-benzofuran-2-on,
2) 5,6-Dihydroxy-[3H]-benzofuran-2-on,
3) 5-Hydroxy-6-methyl-[3H]-benzofuran-2-on,

4) 5-Hydroxy-6-methoxy-[3H]-benzofuran-2-on,
5) 5-Hydroxybenzo-[1,3]-dioxol-2-on,
6) 5-Hydroxybenzo-[1,3]-dioxol-2-thion,
7) 5-Hydroxybenz-[1,3]-oxazol-2-on,
8) 5-Hydroxybenz-[1,3]-oxazol-2-thion,
9) 6-Hydroxybenz-[1,3]-oxazol-2-on,
10) 5-Hydroxybenzimidazol-2-on,
11) 5-Hydroxybenz-[1,3]-oxathiol-2-on,
12) 5-Hydroxybenz-[1,3]-oxathiol-2-onimin, und
13) 5-Hydroxybenz-[1,3]-oxathiol-2-thion.

Nachstehend werden beispielsweise einige Literaturstellen zur Herstellung verschiedener von diesen Verbindungen angegeben.

1) 5-Hydroxy-[3H]-benzofuran-2-on
— Beilsteins „Handbuch der Organischen Chemie", IV. Auflage (1975), Bd. 18, S. 155
3) 5-Hydroxy-6-methyl-[3H]-benzofuran-2-on
— Beilsteins „Handbuch der Organischen Chemie", IV. Auflage (1975), Bd. 18, S. 174
5) 5-Hydroxybenzo-[1,3]-dioxol-2-on
— Beilsteins „Handbuch der Organischen Chemie", IV. Auflage (1977), Bd. 19, S. 2508 und modifiziert nach „Chemische Berichte", 91 (1958), S. 1314
7) 5-Hydroxybenz-[1,3]-oxazol-2-on
— „Chemische Berichte", 93 (1960), S. 1331 ff.
9) 6-Hydroxybenz-[1,3]-oxazol-2-on
— „Chemische Berichte", 93 (1960), S. 1331 ff.
10) 5-Hydroxybenzimidazol-2-on
— Beilsteins „Handbuch der Organischen Chemie", IV. Auflage (1981), Bd. 25, S. 85
11) 5-Hydroxybenz-[1,3]-oxathiol-2-on
— „Methoden der Organischen Chemie" (Houben-Weyl), IV. Auflage (1977), Bd. VII/3a, S. 664
— Beilsteins „Handbuch der Organischen Chemie", IV. Auflage (1977), Bd. 19, S. 2510
— „Chemische Berichte", 95 (1962), S. 1771 ff.
12) 5-Hydroxybenz-[1,3]-oxathiol-2-onimin
— „Methoden der Organischen Chemie "(Houben-Weyl), IV. Auflage (1977), Bd. VII/3a, S. 664
— „Chemische Berichte", 95 (1962), S. 1771 ff.
13) 5-Hydroxybenz-[1,3]-oxathiol-2-thion
— „Chemische Berichte", 95 (1962), S. 1771 ff.

Weitere Literaturstellen für die Herstellung von Verbindungen, die unter die oben angegebene allgemeine Formel I fallen, sind beispielsweise die folgenden:

7-Brom-5-hydroxy-[3H]-benzofuran-2-on
($X=CH_2$, Y=O, Z=O, R=Halogen)
— Beilsteins „Handbuch der Organischen Chemie", IV. Auflage (1975), Bd. 18, S. 155
5-Hydroxyindolin-2-on
($X=CH_2$, Y=NH, Z=O, R=H)
— Beilsteins „Handbuch der Organischen Chemie", IV. Auflage (1979), Bd. 21, S. 6079
3-Alkyl-5-hydroxybenzoxazolinon
(X=N−R', Y=O, Z=O, R=H)
— Roczniki Chemii, „Ann. Soc. Chim. Polonorum", 48, 1065 (1974) und polnisches Patent Nr. 85785
6-Hydroxy-2-oxo-[1,3]-benzoxathiol
(X=O, Y=S, Z=O, R=H)
— „Chemische Berichte", 95 (1962), S. 1771

Darüber hinaus können die Haarfärbemittel der vorliegenden Anmeldung als Farbstoffvorstufen insbesondere natürlich vorkommende, aber auch andere geeignete aromatische Verbindungen, die eine Hydroxygruppe im Molekül aufweisen und zusätzlich ein Stickstoffatom oder mehrere Stickstoffatome im Molekül haben können, enthalten.

Beispiele für solche Verbindungen sind

a) aromatische Verbindungen mit mindestens einer Hydroxygruppe im Molekül wie Orcin, Hydrochinon, Pyrogallol, Hydroxyhydrochinon, Protocatechualdehyd, Thymol, Gujacol, Arbutin, Brenzkatechin, Juglon, Lawson, Flavonoide, Grevelline, Derivate der Salicylsäure, der Kaffeesäure, der Chlorogensäure, der Pulvinsäure, des Hydroxycumarins, sowie

b) aromatische Verbindungen mit mindestens einer Hydroxygruppe und zusätzlich mindestens einem Stickstoffatom im Molekül wie 3-Hydroxyanthranilsäure, Kynurensäure, Xanthurensäure, Dopa, Tyrosin, Derivate der Anthranilsäure, der Nicotinsäure, der Isonicotinsäure, des Pyrrols, der Picolinsäure, des Kynurenins, der Ommatine, der Pyrimidine und der Purine.

Diese Verbindungen können in einem Verhältnis von 0,1 bis 1 mol, bezogen auf 1 mol der als Farbstoffvorstufen enthaltenen Verbindungen der allgemeinen Formel I, eingesetzt werden.

Die Gesamtmenge der in den erfindungsgemässen Haarfärbemitteln enthaltenen, als Farbstoffvorstufen dienenden Verbindungen, beträgt zweckmässigerweise 0,1 bis 5,0, vorzugsweise 1 bis 4 Gewichtsprozent.

Ausserdem ist es möglich, dass die Haarfärbemittel noch zusätzlich übliche direktziehende Haarfarbstoffe enthalten.

Zur Erhöhung der Farbtiefe der Haarfärbungen können in den Haarfärbemitteln gemäss dieser Anmeldung darüber hinaus natürlich vorkommende Aminosäuren, deren Ester mit niederen Alkoholen von 1 bis 5 Kohlenstoffatomen und/oder deren Amide sowie Mono- oder Dialkylamide enthalten sein.

Beispiele hierfür sind die Aminosäuren Glycin, Prolin, Hydroxyprolin, Serin, Cystein, Histidin und Tryptophan, deren Ester mit niederen Alkoholen sowie deren Amide bzw. Mono- oder Dialkylamide, wobei die Alkylgruppe an Amidstickstoff 1 bis 5 Kohlenstoffatome aufweist.

Die Aminosäuren und ihre genannten Derivate können in den Mitteln insbesondere in einem Verhältnis von 0,05 bis 1 mol, bezogen auf 1 mol der darin als Farbstoffvorstufen vorliegenden Verbindungen, enthalten sein.

Zur Beschleunigung der Oxidation der in den erfindungsgemässen Haarfärbemitteln als Farbstoffvorstufen enthaltenen Verbindungen können diese Mittel zusätzlich geringe Mengen von Redoxkatalysatoren, insbesondere Glycinkupfer, in einer Menge von 0,01 bis 0,1 Gew.% enthalten.

Schliesslich können in den Haarfärbemitteln noch weitere übliche kosmetische Zusätze, beispielsweise Antioxidantien wie Ascorbinsäure oder Natriumsulfit, Alkalisierungsmittel wie Alkalihydroxide, Ammonium- bzw. Alkalicarbonat und Ammonium- bzw. Alkalihydrogencarbonat, organische Säuren wie z.B. Essigsäure, Milchsäure und Zitronensäure, Lösungsmittel, Parfüm, Quellmittel, Netzmittel, Emulgatoren, Verdicker, Pflegestoffe und andere vorhanden sein.

Die Zubereitungsform kann die einer Lösung, vorzugsweise einer Creme, eines Gels oder einer Emulsion sein. Ihre Zusammensetzung stellt eine Mischung der Farbstoffkomponenten mit den für solche Zubereitungen üblichen Bestandteilen dar. Als übliche Bestandteile von Cremes, Gelen oder Emulsionen kommen beispielsweise Netzmittel oder Emulgatoren aus den Klassen der anionischen, kationischen oder nichtionogenen oberflächenaktiven Substanzen wie Fettalkoholsulfate, Alkylsulfonate, Alkylbenzolsulfonate, Alkyltrimethylammoniumsalze, oxäthylierte Fettalkohole, oxäthylierte Nonylphenole, Fettsäurealkanolamide, ferner Verdicker wie höhere Fettalkohole, Stärke, Cellulosederivate, Paraffinöl und Fettsäuren sowie ausserdem Pflegestoffe wie Lanolinderivate, Cholesterin und Pantothensäure in Betracht. Die erwähnten Bestandteile werden in den für solche Zwecke üblichen Mengen verwendet, z.B. die Netzmittel und Emulgatoren in Konzentrationen von 0,5 bis 30 Gew.%, während die Verdicker in einer Menge von etwa 0,1 bis 25 Gew.% in den Zubereitungen enthalten sein können.

Je nach Zusammensetzung können die erfindungsgemässen Färbemittel schwach sauer, neutral oder alkalisch reagieren. Insbesondere weisen sie im sauren Bereich einen pH-Wert zwischen 2 und 4 und im alkalischen Bereich einen pH-Wert zwischen 7 und 8,5 auf, wobei die Einstellung vorzugsweise mit einer organischen Säure bzw. mit Ammoniak oder Ammoniumhydrogencarbonat erfolgt. Für die Einstellung eines alkalischen pH-Wertes können aber auch organische Amine, z.B. Mono- oder Triäthanolamin, Verwendung finden.

Bei dem erfindungsgemässen Verfahren zur oxidativen Färbung von Haaren geht man in der Weise vor, dass man im ersten Schritt ein vorzugsweise schwach sauer oder alkalisch eingestelltes Haarfärbemittel, das als Farbstoffvorstufe mindestens eine Verbindung der allgemeinen Formel

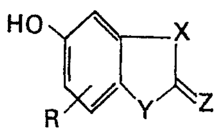 (I)

enthält, worin

X = $CH_2$, O, S, NH, NR', CH(OH), CHR', $CH(NH_2)$, $CH(CH_3)$,

Y = O, NH,

Z = O, S, NH,

R = H, OH, R', OR', $NR'_2$, $NO_2$, Halogen, und

R' = $C_1$ bis $C_5$-Alkyl

bedeuten, gleichmässig auf das Haar aufträgt, etwa 10 bis 20 min bei einer Temperatur von 20 bis 40° C einwirken lässt, im zweiten Schritt a) wenn das Haarfärbemittel alkalisch eingestellt ist, eine 1 bis 8%ige alkalische Lösung eines geeigneten Oxidationsmittels, vorzugsweise von Wasserstoffperoxid oder Ammoniumperoxodisulfat, aufbringt, und weitere 10 bis 20 min bei gleicher Temperatur auf das Haar einwirken lässt, b) wenn das Haarfärbemittel sauer eingestellt ist, eine entsprechende neutrale Oxidationsmittellösung auf das Haar aufbringt, etwa 5 bis 15 min einwirken lässt, gegebenenfalls im Anschluss hieran ein Alkalisierungsmittel, vorzugsweise in Form einer 5 bis 10%igen Ammoniaklösung, aufträgt und etwa 15 min einwirken lässt, sodann im dritten Schritt das Haar bei vorangegangener Anwendung einer alkalischen Lösung zunächst mit einer wässerigen Lösung einer schwachen organischen Säure, vorzugsweise der Zitronensäure, die gegebenenfalls EDTA*) enthält, spült und schliesslich in allen Fällen mit Wasser auswäscht.

Von besonderer Bedeutung ist hierbei der durch die Verwendung von Verbindungen der allgemeinen Formel

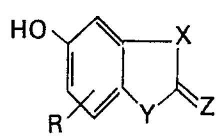 (I)

worin

X = $CH_2$, O, S, NH, NR', CH(OH), CHR', $CH(NH_2)$, $CH(CH_3)$,

Y = O, NH,

Z = O, S, NH,

R = H, OH, R', OR', $NR'_2$, $NO_2$, Halogen, und

R' = $C_1$ bis $C_5$-Alkyl

bedeuten, als Farbstoffvorstufe in den hier beschriebenen Haarfärbemitteln in toxikologischer und dermatologischer Hinsicht erzielte technische Fortschritte; dies gilt insbesondere im Vergleich mit solchen bekannten, als Farbstoffvorstufe dienenden Verbindungen, die eine Aminogruppe im Molekül aufweisen. So wird beispielsweise das 5-Hydroxy-[3H]-benzofuran-2-on bei einer Resorption in den Körper durch Hydrolyse in die im menschlichen Stoffwechsel ohnehin vorkommende und toxikologisch unbedenkliche Homogentisinsäure überführt.

Die erfindungsgemässen Haarfärbemittel ergeben zudem natürlich wirkende und stabile Färbungen, wobei im allgemeinen tiefere Farbnuancen erreicht werden als bei Verwendung von Di- oder Polyphenolen.

Die nachstehenden Beispiele sollen den Gegenstand der Erfindung näher erläutern.

*Beispiel 1*

1,60 g 5-Hydroxy-[3H]-benzofuran-2-on
0,80 g Protocatechualdehyd
0,40 g Glycinamid
0,40 g Tryptophanmethylester
0,40 g Dopa
0,03 g Glycinkupfer
1,00 g Hydroxyäthylcellulose, mittelviskos →

---

*) EDTA = Äthylendiamintetraacetat

9,50 g Guanidinhydrochlorid
2,00 g Ammoniumhydrogencarbonat
40,00 g Äthanol
43,87 g Wasser
100,00 g

Dieses Haarfärbemittel, das einen pH-Wert von pH = 7,8 aufweist, wird auf unbehandelte blonde menschliche Haare aufgetragen und dort 15 min lang bei einer Temperatur von 25° C einwirken gelassen. Anschliessend werden 50 ml einer wässerigen Lösung von 6 Gew.% Ammoniak und 1,5 Gew.% Wasserstoffperoxid auf das Haar aufgebracht. Diese Lösung wird ebenfalls 15 min lang bei gleicher Temperatur einwirken gelassen. Danach wird das Haar zunächst mit einer 10%igen Zitronensäurelösung, die zusätzlich 0,05 Gew.% EDTA enthält, gespült und schliesslich mit Wasser nachgespült. Das Haar hat eine braun-orange Färbung erhalten.

*Beispiel 2*

1,60 g 5-Hydroxy-[3H]-benzofuran-2-on
0,40 g Anthranilsäuremethylester
0,40 g Glycinmethylester
0,40 g Tryptophanmethylester
0,03 g Glycinkupfer
1,00 g Hydroxyäthylcellulose, mittelviskos
9,50 g Guanidinhydrochlorid
2,00 g Essigsäure
40,00 g Äthnol
44,67 g Wasser
100,00 g

Man bringt dieses gelförmige Haarfärbemittel auf gebleichte menschliche Haare auf und lässt es 15 min lang bei einer Temperatur von 25° C einwirken. Anschliessend werden 50 ml einer 5%igen Ammoniumperoxodisulfatlösung gleichmässig auf dem Haar verteilt und weitere 15 min lang bei gleicher Temperatur einwirken gelassen. Schliesslich wird das Haar mit Wasser sorgfältig ausgespült. Das Haar hat eine helle grau-braune Färbung erhalten.

*Beispiel 3*

Ein Haarfärbemittel der in Beispiel 2 angegebenen Zusammensetzung wird auf gebleichte menschliche Haare aufgebracht und 15 min lang bei einer Temperatur von 25° C einwirken gelassen. Anschliessend werden 50 ml einer 5%igen Ammoniumperoxodisulfatlösung aufgebracht und 10 min einwirken gelassen. Hierauf gibt man 20 ml einer 9%igen Ammoniaklösung auf das Haar und lässt diese 15 min lang einwirken. Schliesslich wird das Haar zunächst mit einer 10%igen wässerigen Zitronensäurelösung, die 0,05% EDTA enthält, gespült und danach mit Wasser nachgespült. Das Haar hat eine kastanienbraune Färbung erhalten.

*Beispiel 4*

3,00 g 5-Hydroxy-benzo-[1,3]-dioxol-2-on
0,80 g Glycinamid
1,00 g Hydroxyäthylcellulose, mittelviskos
9,50 g Cuanidinhydrochlorid
0,03 g Glycinkupfer
2,00 g Ammoniumhydrogencarbonat
25,00 g Äthanol
58,67 g Wasser
100,00 g

Dieses Haarfärbemittel, das mit Ammoniumcarbonatlösung auf einen pH-Wert von pH=7,5 eingestellt wurde, trägt man auf hellblonde menschliche Haare auf und lässt es 15 min bei einer Temperatur von 25° C einwirken. Anschliessend werden 50 ml einer wässerigen Lösung von 3 Gew.% Ammoniak und 5 Gew.% Ammoniumperoxodisulfat auf das Haar aufgebracht. Man lässt 15 min lang bei gleicher Temperatur einwirken. Danach wird das Haar zunächst mit einer 10%igen Zitronensäurelösung, die zusätzlich 0,05 Gew.% EDTA enthält, gespült und schliesslich mit Wasser nachgespült. Das Haar hat eine dunkel-schwarzbraune Färbung erhalten.

*Beispiel 5*

1,60 g 5-Hydroxybenz-[1,3]-oxazol-2-on
1,60 g 5-Hydroxybenz-[1,3]-oxathiol-2-on
0,80 g Orcin
0,40 g Glycinamid
0,40 g Cysteinmethylester
1,00 g Hydroxyäthylcellulose, mittelviskos
9,50 g Guanidinhydrochlorid
0,03 g Glycinkupfer
2,00 g Ammoniumhydrogencarbonat
82,67 g Wasser
100,00 g

Dieses Haarfärbemittel, das einen pH-Wert von pH=7,6 aufweist, wird auf gebleichtes Haar aufgetragen und 15 min bei einer Temperatur von 25° C einwirken gelassen. Danach werden 50 ml einer wässerigen Lösung mit einem Gehalt an 6 Gew.% Ammoniak und 1,5 Gew.% Wasserstoffperoxid aufgetragen und ebenfalls 15 min einwirken gelassen. Schliesslich spült man das Haar zunächst mit einer 10%igen Zitronensäurelösung, die 0,05 Gew.% EDTA enthält, und anschliessend mit Wasser aus. Das Haar hat eine goldblonde Färbung erhalten.

Alle in der vorliegenden Anmeldung angegebenen Prozentzahlen stellen Gewichtsprozente dar.

## Patentansprüche

1. Mittel zur oxidativen Färbung von Haaren, dadurch gekennzeichnet, dass es als Farbstoffvorstufe mindestens eine Verbindung der allgemeinen Formel

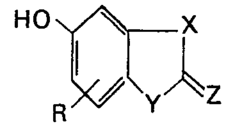

(I)

enthält, worin

X = $CH_2$, O, S, NH, NR', CH(OH), CHR', $CH(NH_2)$, $CH(CH_3)$,

Y = O, NH,
Z = O, S, NH,
R = H, OH, R', OR', $NR'_2$, $NO_2$, Halogen, und
R' = $C_1$ bis $C_5$-Alkyl
bedeuten.

2. Mittel nach Anspruch 1, dadurch gekennzeichnet, dass es als Farbstoffvorstufe mindestens eine der Verbindungen

5-Hydroxy-[3H]-benzofuran-2-on,
5,6-Dihydroxy-[3H]-benzofuran-2-on,
5-Hydroxy-6-methyl-[3H]-benzofuran-2-on,
5-Hydroxy-6-methoxy-[3H]-benzofuran-2-on,
5-Hydroxybenzo-[1,3]-dioxol-2-on,
5-Hydroxybenzo-[1,3]-dioxol-2-thion,
5-Hydroxybenz-[1,3]-oxazol-2-on,
5-Hydroxybenz-[1,3]-oxazol-2-thion,
6-Hydroxybenz-[1,3]-oxazol-2-on,
5-Hydroxybenzimidazol-2-on,
5-Hydroxybenz-[1,3]-oxathiol-2-on,
5-Hydroxybenz-[1,3]-oxathiol-2-onimin, und
5-Hydroxybenz-[1,3]-oxathiol-2-thion

enthält.

3. Mittel nach Anspruch 1, dadurch gekennzeichnet, dass es als Farbstoffvorstufen Verbindungen gemäss Formel (I) in einer Konzentration von insgesamt 0,1 bis 5 Gew.%, vorzugsweise 1 bis 4 Gew.%, enthält.

4. Mittel nach den Ansprüchen 1 bis 3, dadurch gekennzeichnet, dass es zusätzlich andere Verbindungen als Farbstoffvorstufen sowie direktziehende Farbstoffe enthält.

5. Mittel nach Anspruch 4, dadurch gekennzeichnet, dass es als Farbstoffvorstufe mindestens eine Verbindung enthält, die ausgewählt ist aus

a) den aromatischen Verbindungen mit mindestens einer Hydroxygruppe im Molekül Orcin, Hydrochinon, Pyrogallol, Hydroxyhydrochinon, Protocatechualdehyd, Thymol, Gujacol, Arbutin, Brenzkatechin, Juglon, Lawson, Flavonoide, Grevelline, sowie Derivaten der Salicylsäure, der Kaffeesäure, der Chlorogensäure, der Pulvinsäure, des Hydroxycumarins, sowie ferner

b) den aromatischen Verbindungen mit mindestens einer Hydroxygruppe und zusätzlich mindestens einem Stickstoffatom im Molekül 3-Hydroxyanthranilsäure, Kynurensäure, Xanthuren säure, Dopa, Tyrosin, den Derivaten der Anthranilsäure, der Nicotinsäure, der Isonicotinsäure, des Pyrrols, der Picolinsäure, des Kynurenins, der Ommatine, der Pyrimidine und der Purine.

6. Mittel nach den Ansprüchen 1 bis 5, dadurch gekennzeichnet, dass die Gesamtmenge der als Farbstoffvorstufen enthaltenen Verbindungen 0,1 bis 5,0 Gew.%, vorzugsweise 1 bis 4 Gew.%, beträgt.

7. Mittel nach den Ansprüchen 1 bis 6, dadurch gekennzeichnet, dass es zusätzlich natürlich vorkommende Aminosäuren, deren Ester mit niederen Alkoholen von 1 bis 5 Kohlenstoffatomen und/oder deren Amide sowie Mono- oder Dialkylamide enthält.

8. Mittel nach den Ansprüchen 1 bis 7, dadurch gekennzeichnet, dass es als Redoxkatalysator Glycinkupfer in einer Menge von 0,01 bis 0,1 Gew.% enthält.

9. Verfahren zur oxidativen Färbung von Haaren, dadurch gekennzeichnet, dass man im ersten Schritt ein vorzugsweise schwach sauer oder alkalisch eingestelltes Haarfärbemittel, das als Farbstoffvorstufe mindestens eine Verbindung der allgemeinen Formel

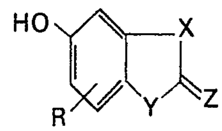

(I)

worin
X = $CH_2$, O, S, NH, NR', CH(OH), CHR', $CH(NH_2)$, $CH(CH_3)$,
Y = O, NH,
Z = O, S, NH,
R = H, OH, R', OR', $NR'_2$, $NO_2$, Halogen, und
R' = $C_1$ bis $C_5$-Alkyl
bedeuten, gleichmässig auf das Haar aufträgt, etwa 10 bis 20 min bei einer Temperatur von 20 bis 40° C einwirken lässt, im zweiten Schritt a) wenn das Haarfärbemittel alkalisch eingestellt ist, eine 1 bis 8%ige alkalische Lösung eines geeigneten Oxidationsmittels, vorzugsweise von Wasserstoffperoxid oder Ammoniumperoxodisulfat, aufbringt, weitere 10 bis 20 min bei gleicher Temperatur auf das Haar einwirken lässt, b) wenn das Haarfärbemittel sauer eingestellt ist, eine entsprechende neutrale Oxidationsmittellösung auf das Haar aufbringt, etwa 5 bis 15 min einwirken lässt, gegebenenfalls im Anschluss hieran ein Alkalisierungsmittel, vorzugsweise in Form einer 5 bis 10%igen Ammoniaklösung, aufträgt und etwa 15 min einwirken lässt, sodann im dritten Schritt das Haar bei vorangegangener Anwendung einer alkalischen Lösung zunächst mit einer wässerigen Lösung einer schwachen organischen Säure, vorzugsweise der Zitronensäure, die gegebenenfalls zusätzlich EDTA enthält, spült und schliesslich in allen Fällen mit Wasser auswäscht.

## Revendications

1. Composition pour la teinture par oxydation des cheveux, caractérisée en ce qu'elle contient en tant que progéniteur du colorant au moins un composé de formule générale:

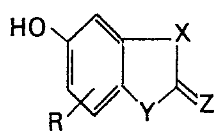

(I)

où:
X = $CH_2$, O, S, NH, NR', CH(OH), CHR', $CH(NH_2)$, $CH(CH_3)$,
Y = O, NH,
Z = O, S, NH,
R = H, OH, R', OR', $NR'_2$, $NO_2$, halogène,
R' = $C_1$ à $C_5$-alcoyle.

2. Composition selon la revendication 1, caractérisée en ce qu'elle contient en tant que progéniteur du colorant au moins l'un des composés suivants:

5-hydroxy-3H-benzofuranne-2-one,
5,6-dihydroxy-3H-benzofuranne-2-one,
5-hydroxy-6-méthyl-3H-benzofuranne-2-one,
5-hydroxy-6-méthoxy-3H-benzofuranne-2-one,
5-hydroxybenzo-[1,3]-dioxol-2-one,
5-hydroxybenzo-[1,3]-dioxol-2-thione,
5-hydroxybenz-[1,3]-oxazol-2-one,
5-hydroxybenz-[1,3]-oxazol-2-thione,
6-hydroxybenz-[1,3]-oxazol-2-one,
5-hydroxybenzimidazol-2-one,
5-hydroxybenz-[1,3]-oxathiol-2-one,
5-hydroxybenz-[1,3]-oxathiol-2-one-imine, et
5-hydroxybenz-[1,3]-oxathiol-2-thione.

3. Composition selon la revendication 1, caractérisée en ce qu'elle contient en tant que progéniteurs de colorants des composés selon la formule (I), selon une concentration d'ensemble de 0,1 à 5% en poids et, de préférence, de 1 à 4% en poids.

4. Composition selon l'une des revendications 1 à 3, caractérisée en ce qu'elle contient, en plus, d'autres composés en tant que progéniteurs de colorants, ainsi que des colorants à action directe.

5. Composition selon la revendication 4, caractérisée en ce qu'elle contient en tant que progéniteur du colorant au moins un composé choisi parmi:

a) les composés aromatiques comprenant au moins un groupe hydroxy dans la molécule orcine, hydroquinone, pyrogallol, hydroxyhydroquinone, aldéhyde protocatéchique, thymol, gaïacol, arbutine, pyrocatéchine, juglon, Lawson, flavonoïde, grevelline, ainsi que des dérivés de l'acide salicylique, de l'acide caféique, de l'acide chlorogénique, de l'acide pulvinique, de l'hydroxycumarine, ainsi qu'en outre:

b) des composés aromatiques comprenant au moins un groupe hydroxy et en plus au moins un atome d'azote dans la molécule 3-hydroxyacide anthranilique, acide cynurénique, acide xanthurénique, dopa, tyrosine, des dérivés de l'acide anthranilique, de l'acide nicotinique, de l'acide isonicotinique, du pyrrole, de l'acide picolinique, de la cynurénine, de l'ommatine, des pyrimidines et des purines.

6. Composition selon l'une des revendications 1 à 5, caractérisée en ce que la quantité totale des composés contenus en tant que progéniteurs de colorants est comprise entre 0,1 et 5,0% en poids et, de préférence, entre 1 et 4% en poids.

7. Composition selon l'une des revendications 1 à 6, caractérisée en ce qu'elle contient, en plus, des acides aminés intervenant naturellement dont les esters avec des alcools faibles contiennent de 1 à 5 atomes de carbone et/ou dont les amides contiennent des mono- aussi bien que dialcoylamides.

8. Composition selon l'une des revendications 1 à 7, caractérisée en ce qu'elle contient, en tant que catalyseur redox, de la glycérine-cuivre selon une quantité comprise entre 0,01 et 0,1% en poids.

9. Procédé de coloration par oxydation des cheveux, caractérisée en ce que l'on applique régulièrement sur les cheveux, au cours d'une première étape, une composition de coloration des cheveux de préférence légèrement acide ou alcaline, contenant en tant que progéniteur du colorant au moins un composé de formule générale:

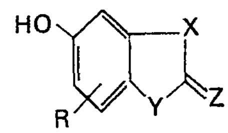
(I)

où:
X = $CH_2$, O, S, NH, NR', CH(OH), CHR', $CH(NH_2)$, $CH(CH_3)$,
Y = O, NH,
Z = O, S, NH,
R = H, OH, R', OR', $NR'_2$, $NO_2$, halogène,
R' = $C_1$ à $C_5$-alcoyle,

qu'on laisse agir pendant 10 à 20 min environ à une température de 20 à 40° C, et en ce que l'on procède à une seconde étape consistant: a) lorsque la composition de coloration est alcaline, en l'application d'une solution alcaline à 1 à 8% d'un agent d'oxydation approprié, et de préférence de peroxyde d'oxygène ou de peroxodisulfate d'ammonium, qu'on laisse agir sur les cheveux pendant 10 à 20 min à la même température; b) lorsque la composition de coloration des cheveux est acide, en l'application sur les cheveux d'une solution d'un agent d'oxydation neutre correspondant, qu'on laisse agir pendant 5 à 15 min environ, éventuellement à la suite un agent d'alcalinisation, de préférence sous la forme d'une solution d'ammoniac de 5 à 10%, qu'on laisse agir pendant 15 min environ; puis, au cours d'une troisième étape, quand on a commencé par utiliser une solution alcaline, on lave avec une solution aqueuse d'un acide organique faible, de préférence de l'acide citrique, contenant éventuellement en plus de l'EDTA, puis on rince à l'eau dans tous les cas.

## Claims

1. Composition for the oxidative colouring of hair, characterised in that it contains, as dye precursor, at least one compound of the general formula:

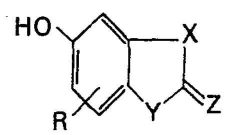
(I)

in which:
X = $CH_2$, O, S, NH, NR', CH(OH), CHR', $CH(NH_2)$, $CH(CH_3)$
Y = O, NH
Z = O, S, NH
R = H, OH, R', OR', $NR'_2$, $NO_2$, halogen
R' = $C_1$ to $C_5$-alkyl

2. Composition according to claim 1, characterised in that it contains, as dye precursor, at least one of the compounds:
5-hydroxy-3H-benzofuran-2-one,
5,6-dihydroxy-3H-benzofuran-2-on,

5-hydroxy-6-methyl-3H-benzofuran-2-one,
5-hydroxy-6-methoxy-3H-benzofuran-2-one,
5-hydroxybenzo-[1,3]-dioxol-2-one,
5-hydroxybenzo-[1,3]-dioxol-2-thione,
5-hydroxybenz-[1,3]-oxazol-2-one,
5-hydroxybenz-[1,3]-oxazol-2-thione,
6-hydroxybenz-[1,3]-oxazol-2-one,
5-hydroxybenzimidazol-2-one,
5-hydroxybenz-[1,3]-oxathiol-2-one,
5-hydroxybenz-[1,3]-oxathiol-2-one-imine, and
5-hydroxybenz-[1,3]-oxathiol-2-thione.

3. Composition according to claim 1, characterised in that it contains, as dye precursors, compounds according to formula (I) in a total concentration from 0.1 to 5 wt.-%, preferable 1 to 4 wt.-%.

4. Composition according to one of claims 1 to 3, characterised in that it additionally contains other compounds as dye precursors as well as direct dyes.

5. Composition according to claim 4, characterised in that it contains, as dye precursor, at least one compound, which is selected from:

a) the aromatic compounds with at least one hydroxyl group in the molecule orcin, hydroquinone, pyrogallol, hydroxyhydroquinone, protocatechic aldehyde, thymol, gujacol, arbutin, pyrocatechin, juglon, Lawson, flavonoides, grevelline, as well as derivatives of salicylic acid, of coffeic acid, of chlorogenic acid, of pulvinic acid, of hydroxycumarine, as well as also

b) the aromatic compounds having at least one hydroxyl group and additionally at least one nitrogen atom in the molecule 3-hydroxyanthranilic acid, kynurenic acid, xanthurenic acid, dopa, tyrosine, derivatives of anthranilic acid, of nicotinic acid, of isonicotinic acid, of pyrrole, of picolinic acid, of kynurenine, of ommatine, of pyrimidines, and purines.

6. Composition according to one of claims 1 to 5, characterised in that the total quantity of the compounds present, as dye precursors, amounts to 0.1 to 5,0 wt.-%, preferably 1 to 4 wt.-%.

7. Composition according to one of claims 1 to 6, characterised in that it additionally contains naturally occurring amino acids, their esters with lower alcohols from 1 to 5 carbon atoms and/or their amides as well as mono- or dialkylamides.

8. Composition according to one of claims 1 to 7, characterised in that it contains, as redox catalyst, glycine-copper in amount from 0.01 to 0.1 wt.-%.

9. Process for the oxidative colouring of hair, characterised in that, in a first step, a preferably weakly acid or alkaline-adjusted hair colouring composition is evenly applied to the hair, which composition contains, as dye precursors, at least one compound of the general formula:

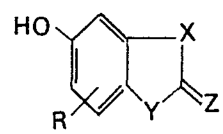

(I)

wherein:

X = $CH_2$, O, S, NH, NR', CH(OH), CHR', $CH(NH_2)$, $CH(CH_3)$

Y = O, NH

Z = O, S, NH

R = H, OH, R', OR', $NR'_2$, $NO_2$, halogen

R' = $C_1$ to $C_5$-alkyl

which composition is allowed to act for about 10 to 20 min at a temperature from 20 to 40° C, in the second step: (a) if the hair colouring medium is alkaline-adjusted, a 1 to 8% alkaline solution of a suitable oxidative medium, preferably hydrogen peroxide or ammonium peroxodisulphate, is applied, allowed to act on the hair for a further 10 to 20 min at the same temperature, (b) if the hair colouring composition is acid-adjusted, a corresponding neutral oxidising agent solution is applied to the hair, is allowed to act for about 5 to 15 min, if desired and in connection therewith an alkylisating agent, preferably in the form of a 5 to 10% ammonia solution, is applied and allowed to act for about 15 min, then, in the third step, hair to which an alkaline solution has previously been applied is washed with an aqueous solution of a weak organic acid, preferably citric acid, which optionally additionally contains EDTA, and finally and in all cases is washed out with water.